# EUROPEAN PATENT SPECIFICATION

(11) **EP 4 259 066 B1**
(45) Date of publication and mention of the grant of the patent: **24.07.2024**
(21) Application number: 21830674.4
(22) Date of filing: 09.12.2021
(51) Int. Cl.: A61K 8/02, A61K 8/04, A61K 8/26, A61K 8/28, A61K 8/92, A61Q 15/00

(54) **AN ANTIPERSPIRANT COMPOSITION**
SCHWEISSHEMMENDE ZUSAMMENSETZUNG
COMPOSITION ANTITRANSPIRATION

(30) Priority: 11.12.2020 IN 202021053948; 02.08.2021 EP 21189217
(43) Date of publication of application: 18.10.2023
(73) Proprietor: Unilever IP Holdings B.V., 3013 AL Rotterdam (NL); Unilever Global IP Limited, Wirral, Merseyside CH62 4ZD (GB)
(72) Inventor: DAS, Somnath, Whitefield, Bangalore 560 066 (IN); NYALAM, Praveen, Whitefield, Bangalore 560 066 (IN)
(74) Representative: Whaley, Christopher
(86) International application number: PCT/EP2021/084932
(87) International publication number: WO 2022/122884

(56) References cited:
- WO-A1-2015/112468
- WO-A1-2021/028180
- US-A1- 2012 244 099
- DATABASE GNPD [online] MINTEL; 5 November 2020 (2020-11-05), ANONYMOUS: "48H Anti-Perspirant Deodorant Roll On", XP055880000, retrieved from https://www.gnpd.com/sinatra/recordpage/8243329/ Database accession no. 8243329
- DATABASE GNPD [online] MINTEL; 2 August 2011 (2011-08-02), ANONYMOUS: "Anti-Perspirant Deodorant Roll-On", XP055880004, retrieved from https://www.gnpd.com/sinatra/recordpage/1600028/ Database accession no. 1600028

## Description

### Field of the invention

The present invention relates to an antiperspirant (AP) composition. The invention more particularly relates to an anhydrous antiperspirant composition that comprises conventional metal-based AP actives and natural oil that ensures that when such compositions are used on a body part e.g., the axilla there is minimal or no yellow coloured staining of the fabric on repeated use of such fabric through several use-wash-rinse-dry cycles. The preferred forms of such products are as an aerosol or in a stick format.

### Background of the invention

The present invention relates to substantially anhydrous compositions, such as those that contain antiperspirant actives. These actives are added to compositions to reduce perspiration on application to the surface of the body, particularly to the underarm regions of the human body viz. the axilla. Antiperspirant actives used so far are typically astringent metal salts such as aluminium or zirconium salts e.g., aluminium chlorohydrates or sesquichlorohydrates. Such compositions also include natural oils which may be unsaturated e.g., sunflower seed oil for delivering skin care benefits. Since these oils are unsaturated, antioxidants are generally added to such compositions to minimize oxidation of such compounds to ensure minimal colour formation. Typically, antioxidants like ascorbic acid, butyl hydroxy toluene (BHT) and other commercially available compounds like Tinogard TT (Pentaerythrityl tetra-di-t-butyl hydroxyhydrocinnamate) or Tinogard Q (Tris -(Tetramethylhydroxy-piperidinol) citrate), both from BASF, have been used. The present inventors have found that when such compositions are applied on the underarm region, the fabric which comes in contact with the composition tends to get stained on repeated use (where the fabric may be washed, rinsed and dried between each use).

The present inventors have found that when a non-ionic surfactant of specific low HLB is included in such anhydrous AP compositions e.g., those in the stick or aerosol format, it tends to minimize this staining problem. Surfactants are generally included in water containing composition to emulsify the oily components. To the knowledge of the present inventors there is no motivation to include non-ionic surfactant in such anhydrous compositions nor have they been added.

The present applicants in WO2020/078665 have solved the problem of staining of fabrics on use of antiperspirant composition which generally contain water and comprising metal based antiperspirant actives and natural oil by using specific phenolic antioxidants. In the present invention they provide the solution to this problem specifically for substantially anhydrous compositions through incorporation of non-ionic surfactants having a specific low HLB value.

Examples where non-ionic surfactants have been included in AP composition have generally been in water containing compositions as an emulsifier or in certain compositions where the non-ionic surfactant has a high HLB value.

US2006115441 (Unilever) discloses in certain examples antiperspirant compositions containing aluminium chlorohydrate, sunflower oil, BHT, non-ionic surfactants stearth 2 and stearth 20 but this is in compositions that contain higher than 60% water.

US2009324317 (Unilever) discloses a dispenser for anhydrous cosmetic products which are configured to minimize photo-destruction of ingredients therein where in an example a composition is disclosed comprising aluminium chlorohydrate, sunflower oil, BHT, and non-ionic surfactants stearth 100 which has an HLB of 18.8.

Thus, there is no known prior art that teaches the solution to the present problem of using low HLB (less than 11) non-ionic surfactant in anhydrous AP compositions.

It is thus an object of the present invention to provide for an anhydrous antiperspirant composition comprising a metal based antiperspirant active and natural oil that minimizes the problem of staining or yellowing of fabrics on repeated use-wash-rinse-dry cycles of the fabric when worn by individuals using such antiperspirant compositions.

### Summary of the invention

According to the first aspect of the present invention there is provided an antiperspirant composition comprising
(a) A metal based antiperspirant active;
(b) A natural oil that comprises a glyceride of an unsaturated carboxylic/fatty acid;
(c) An antioxidant compound;
(d) A non-ionic surfactant having an HLB of less than 11; and
(e) less than 10 wt% water, wherein the non-ionic surfactant is selected from polyoxyethylene sorbitan alkyl esters (sold as Tween surfactants), fatty alcohol ethoxylates (sold as Brij surfactants), or sorbitan monoester (sold as Span surfactants), alkyl polyglucosides or C8-C16 fatty alcohol glycoside.

According to another aspect of the present invention there is provided a method of minimizing staining or yellowish coloration of fabric worn by an individual comprising the steps of (a) applying a composition of the invention on to a body part preferably the axilla of the individual followed by (b) washing, (c) rinsing and (d) drying the fabric.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The compositions of the invention are typically "personal care compositions", suitable for use as detailed below. Further, use of the compositions of the invention is typically for cosmetic or non-therapeutic use.

In some embodiments of the present invention, the compositions may be used for the therapeutic treatment of hyperhidrosis (extreme sweating).

By "An Antiperspirant Composition" as used herein, is meant to include a composition for topical application to the skin of mammals, especially humans. Such a composition is preferably of the leave-on type. By a leave-on composition is meant a composition that is applied to the desired skin surface and left on for a period of time (say from one minute to 24 hours) after which it may be wiped or rinsed off with water, usually during the regular course of personal washing. The composition may also be formulated into a product which is applied to a human body for improving the appearance, cleansing, odor control or general aesthetics. The composition of the present invention can be in the form of a liquid, foam, spray, aerosol, gel or stick form. "Skin" as used herein is meant to include skin on any part of the body (e.g., neck, chest, back, arms, underarms, hands, legs, buttocks and scalp) especially the underarms.

The composition of the invention is substantially anhydrous. By "anhydrous" is meant that the composition comprises no added water. However, the composition may comprise some water that gets included in the composition from the ingredients formulated to form the composition. The water content may also be as a result of the ingress of water from the moisture in the atmosphere which reaches an equilibrium with the components of the composition. In effect, the composition comprises less than less than 10 wt.%, preferably less than 5 wt.%, furthermore preferably less than 2 wt.%, and most preferably less than 0.5 wt.% by weight of the composition.

The anti-perspirant composition according to this invention includes a metal based antiperspirant active. This may be selected from an aluminium, zirconium or mixed aluminium/zirconium salts, preferably, aluminium chlorohydrate, aluminum-zirconium tetrachlorohydrex glycine complex, aluminum-zirconium octachlorohydrex glycine complex, aluminum-zirconium pentachlorohydrate, aluminum sesquichlorohydrate or mixtures thereof.

Antiperspirant actives for use herein are selected from aluminium, zirconium and mixed aluminium/zirconium salts, including both inorganic salts, salts with organic anions and complexes. Particularly preferred astringent salts are halohydrate salts, and especially chlorohydrate salts, optionally activated. For aerosol compositions, the antiperspirant active is preferably free from zirconium.

Aluminium halohydrates are usually defined by the general formula Al₂(OH)ₓQ_{y}.wH₂O in which Q represents a halogen selected from the group consisting of chlorine, bromine iodine or mixtures thereof, x is variable from 2 to 5 and x + y = 6 while wH₂O represents a variable amount of hydration. Especially effective aluminium halohydrate salts, known as activated aluminium chlorohydrates, are described in EP-A-6739 (Unilever NV et al), the contents of which specification is incorporated herein by reference.

The term aluminium chlorohydrate herein encompasses materials with specified figures for x and y, such as aluminium sesquichlorohydrate and materials in which the chlorohydrate is present as a complex. It will be recognized that alternative names are sometimes used to indicate the presence of hydroxyl substitution, including aluminium hydroxy chloride, aluminium oxychloride or basic aluminium chloride.

Zirconium astringent salts for employment herein as the metal based antiperspirant active can usually be represented by the empirical general formula: ZrO(OH)_{2n-nz}B_{z}.wH₂O in which z is a variable in the range of from 0.9 to 2.0 so that the value 2n-nz is zero or positive, n is the valency of B, and B is selected from the group consisting of chloride, other halide, sulphamate, sulphate or mixtures thereof. Possible hydration to a variable extent is represented by wH₂O. Preferably, B represents chloride. Preferably, the variable z lies in the range from 1.5 to 1.87. In practice, such zirconium salts are commonly not employed by themselves, but as a component of a combined aluminium and zirconium-based antiperspirant active.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed as metal based antiperspirant active. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH₂(NH₂)COOH. Certain of those Al/Zr complexes are commonly called ZAG in the literature. ZAG actives generally contain aluminium, zirconium and chloride with an Al/Zr ratio in a range from 2 to 10, especially 2 to 6, an AI/CI ratio from 2.1 to 0.9 and a variable amount of glycine. Actives of this preferred type are available from Westwood, from Summit and from Reheis. Alternatively, the complex can be preformed with a polyhydric aliphatic alcohol, such as propylene glycol or glycerol. A complex with a chlorohydrate is commonly referred to as a chlorhydrex.

Mixtures of two or more astringent salts can be employed, but, however, it is particularly preferred to employ astringent salts that are free from zirconium, such as aluminium chlorohydrates and so-called activated aluminium chlorohydrates.

According to an especially preferred aspect of the present invention the antiperspirant active is aluminium chlorohydrate, aluminum sesquichlorohydrate or mixtures thereof. The metal based antiperspirant active preferably is included in 1 wt.% to 50 wt.%, more preferably 2 wt.% to 25 wt.%, most preferably 2 wt.% to 15 wt.% by weight of the composition.

The antiperspirant composition comprises a natural oil. The natural oil is preferably selected from at least one of coriander seed oil, borage seed oil, evening primrose oil, maize corn oil, sunflower oil, safflower oil, coconut oil, algal oil or mixtures thereof. More preferred natural oils are sunflower oil, algal oil or coconut oil preferably sunflower oil. The natural oil is preferably included in an amount ranging from 1 wt.% to 10 wt.%, more preferably 1 wt. to 5 wt.% by weight of the composition.

The antiperspirant composition includes an antioxidant compound. Suitable and conventional antioxidants which are generally included in such compositions may be added in the present invention. Such antioxidants are preferably selected from one or more of butyl hydroxy toluene or its derivatives, pentaerythrityl tetra-di-t-butyl hydroxyhydrocinnamate (sold as Tinogard TT), or dilauryl thiodipropionate (sold as Tinogard DA), Tetramethylhydroxypiperidinol) Citrate (sold as Tinogard Q), tocopherol acetate or ascorbic acid and its derivatives. The antiperspirant composition preferably comprises 0.001 wt.% to 10 wt.%, more preferably 0.01 wt.% to 5 wt.%, and most preferably 0.01 wt.% to 2 wt.% antioxidant by weight of the composition.

The composition of the invention includes a non-ionic surfactant which is selected from polyoxyethylene sorbitan alkyl esters (sold as Tween surfactants), fatty alcohol ethoxylates (sold as Brij surfactants), or sorbitan monoester (sold as Span surfactants), alkyl polyglucosides or C₈-C₁₆ fatty alcohol glycoside; having an HLB of less than 11.0.

Thus, any non-ionic surfactant which has an HLB value less than 11.0 may be used in the composition of the invention.

HLB is the hydrophilic lipophilic balance which is calculated using the Griffin method wherein HLB = 20 x Mh / M wherein Mh is the molecular mass of the hydrophilic portion of the molecule and M is the molecular mass of the whole molecule, giving a result on an arbitrary scale of 0 to 20.

A non-exhaustive list of non-ionic surfactants which have a HLB value of less than 11 is given below with the HLB values in the bracket.

Glycol Distearate (1), Sorbitan Trioleate (1.8), Propylene Glycol Isostearate (2.5), Glycol Stearate (2.9), Sorbitan Sesquioleate (3.7), Glyceryl Stearate (3.8), Lecithin (4), Sorbitan Oleate (4.3), Sorbitan Monostearate NF (4.7 ), Sorbitan Stearate (4.7), Sorbitan Isostearate (4.7), Steareth-2 (4.9), Oleth-2 (4.9), Glyceryl Laurate (5.2), Ceteth-2 (5.3), PEG-30 Dipolyhydroxystearate (5.5), Glyceryl Stearate SE (5.8), Sorbitan Stearate (and) Sucrose Cocoate (6), PEG-4 Dilaurate (6), Methyl Glucose Sesquistearate (6.6), PEG-8 Dioleate (8), Sorbitan Laurate (8.6), PEG-40 Sorbitan Peroleate (9), Laureth-4 (9.7), PEG-7 Glyceryl Cocoate (10), PEG-20 Almond Glycerides (10), PEG-25 Hydrogenated Castor Oil (10.8).

The most preferred non-ionic surfactant for use in the present invention is selected from Polyethylene glycol dodecyl ether (sold as Brij L4), Steareth-2 (sold as Brij S2), tri(oxyethylene) dodecyl ether (sold as EO3) or a combination of EOS and hepta (oxyethylene) dodecyl ether (sold as EO7). The non-ionic surfactant is preferably included in 0.5 wt. to 5 wt.% by weight of the composition.

Without wishing to be bound by theory, the inventors believe that the hydrophilic-lipophilic balance (HLB) of a surfactant plays an important role in solubilization of oil during detergency due to lowering of interfacial tension and the oil/ water/ fibre contact point. Low HLB surfactants are expected to solubilize oils efficiently compared to high HLB ones which in interaction with the certain of the ingredients in the composition provides the benefits of the invention.

The composition of the invention is preferably delivered as a product in aerosol form or in stick form.

The composition of the invention preferably comprises a topically acceptable carrier which in the present invention is anhydrous. To enable this, the anhydrous carrier preferably comprises a silicone compound, an alcohol or a wax. The alcohol, when used, could be a low boiling (C₂ to C₄) alcohol or a polyhydric alcohol, preferably a polyhydric alcohol.

The composition of the invention preferably comprises a polyhydric alcohol. Polyhydric alcohol is also referred to in short as polyol. A polyhydric alcohol according to the present invention is a compound having two or more hydroxyl groups. Suitable class of polyhydric alcohols that may be included in the composition of the invention are monomeric polyols, polyalkylene glycols or sugars. Preferred monomeric polyols are glycol; alkylene glycol e.g., propylene glycol; glycerol; or xylitol, more preferably propylene glycol.

Suitable polyalkylene glycols are polyethylene glycol or polypropylene glycol. Sugars for inclusion in the invention could be monomeric, dimeric, trimeric or of the polymeric form. Preferred sugars include glucose, fructose, mannose, sucrose, threitol, erythritol, sorbitol, mannitol, galactitol, adonitol, dextran, or cyclodextrin. Of these the more preferred sugars are glucose, fructose, sucrose, sorbitol, mannitol, adonitol, dextran, or cyclodextrin.

Other components commonly included in conventional antiperspirant compositions may also be incorporated in the composition of the present invention. Such components include skin care agents such as emollients, humectants and skin barrier promoters; skin appearance modifiers such as skin lightening agents and skin smoothing agents; anti-microbial agents, in particular organic anti-microbial agents, and preservatives.

The antiperspirant composition can be applied cosmetically and topically to the skin, broadly speaking, by one of two methods. Some consumers prefer one method and some others, the other method. In one method, sometimes called a contact method, a composition is wiped across the surface of the skin, depositing a fraction of the composition as it passes. In the second method, sometimes called the non-contact method, the composition is sprayed from a dispenser held proximate to the skin, often in an area of about 10 cm² to 20 cm². The spray can be developed by mechanical means of generating pressure on the contents of the dispenser, such as a pump or a squeezable sidewall or by internally generated pressure arising from a fraction of a liquefied propellant volatilizing, the dispenser commonly being called an aerosol.

There are broadly speaking two classes of contact compositions, one of which is liquid and usually applied using a roll-on dispenser or possibly absorbed into or onto a wipe, and in the second of which the antiperspirant active is distributed within a carrier liquid that forms a continuous phase that has been gelled. In one variation, the carrier fluid comprises a solvent for the antiperspirant and in a second variation, the antiperspirant remains a particulate solid that is suspended in an oil, usually a blend of oils.

### Stick or soft solid compositions

Many different materials have been proposed as gellant for a continuous oil phase, including waxes, small molecule gelling agents and polymers. They each have their advantages and of them, one of the most popular class of gellant has comprised waxes, partly at least due to their ready availability and ease of processing, including in particular linear fatty alcohol wax gellants. A gelled antiperspirant composition is applied topically to skin by wiping it across and in contact with the skin, thereby depositing on the skin a thin film.

The nature of the film depends to a significant extent on the gellant that is employed. Although wax fatty alcohols have been employed as gellant for many years, and are effective for the purpose of gelling, the resultant product is rather ineffective at improving the visual appearance of skin, and in particular underarm skin, to which the composition has been applied. This problem has been solved by including ameliorating materials for example, di or polyhydric humectants and/or a triglyceride oil.

### Roll-on

Liquid compositions that are applicable from a roll-on broadly speaking can be divided into two classes, namely those in which an antiperspirant active is suspended in a hydrophobic carrier, such as a volatile silicone and those in which the antiperspirant active is dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. Since the present invention involves a hydrophobic composition, the former is used. The former is very popular because ethanol is a mild bactericide in its own right

### Aerosol compositions

The antiperspirant composition may be delivered through an aerosol composition which comprises a propellant in addition to the other ingredients described hereinabove. Commonly, the propellant is employed in a weight ratio to the base formulation of from 95:5 to 5:95. Depending on the propellant, in such aerosol compositions the ratio of propellant to base formulation is normally at least 20:80, generally at least 30:70, particularly at least 40:60, and in many formulations, the weight ratio is from 90:10 to 50:50. A ratio range of from 70:30 to 90:10 is sometimes preferred.

Propellants herein generally are one of three classes; i) low boiling point gasses liquifided by compression, ii) volatile ethers and iii) compressed non-oxidising gases.

Class i) is conveniently a low boiling point material, typically boiling below -5°C, and often below -15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutane. The second class of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses. The third class of propellant comprises compressed non-oxidising gasses, and in particular carbon dioxide or nitrogen. Inert gases like neon are a theoretical alternative.

The composition of the present invention can comprise a wide range of other optional components. The CTFA Personal care Ingredient Handbook, Second Edition, 1992, which is incorporated by reference herein in its entirety, describes a wide variety of non-limiting personal care and pharmaceutical ingredients commonly used in the skin care industry, which are suitable for use in the compositions of the present invention. Examples include binders, biological additives, buffering agents, colorants, thickeners, polymers, astringents, fragrance, conditioners, exfoliating agents, pH adjusters, preservatives, natural extracts, essential oils, skin sensates, skin soothing agents, and skin healing agents.

According to another aspect of the present invention there is provided a method of minimizing staining or yellowish colouration of fabric comprising the steps of (a) applying a composition of the invention preferably the axilla of an individual followed by (b) washing, (c) rinsing and (d) drying the fabric.

The composition is preferably applied on the axilla. The method is also preferably non-therapeutic or for cosmetic application.

The invention will now be illustrated with the help of the following non-limiting examples.

### Examples

Anti-perspirant aerosol composition as shown in Table 1 below were prepared:

**Table -1**

| Ingredient | wt% |
|---|---|
| Cyclopentasiloxane | 2.5 |
| PPG 14 Butyl ether | 3 |
| Sunflower seed oil | 0.5 |
| BHT | 0.1 |
| Aluminium chlorohydrate | 5.5 |
| Disteardimonium hectorite | 0.6 |
| Propylene carbonate | 0.015 |
| C12-15 alkyl benzoate | 0.5 |
| Octyldodecanol | 0.12 |
| Nonionic surfactant, Type and concentration | See Tables below; 2 |
| Butane, isobutane, propane | To 100 |

### Examples A, B, 1-3: Effect of HLB of the non-ionic surfactant

The effect of inclusion of different types of non-ionic surfactants as in Table -2 in the composition as give in Table - 1 was studied, and the cleaning efficacy was measured as given in the protocol given below. A measure of yellowing Δb* is also given in Table -2 after 2 wash cycles.

### Protocol used for the evaluation

In this study, a 10 X 10 cm² fabric swatch was taken. The central circular area corresponding to 33 cm² was applied with 0.3 g of APA base formulation containing 0.05 g model sebum mix. Study was done with five replicates. The treated swatches were incubated in a hot air oven at 45 °C for 12 h.

Treated fabrics were washed in an IFB front loader washing machine, programmed for wash and two rinses. Treated fabrics were stapled to clean, air dry polycotton ballast to obtain an approximate load of 2 ±0.1 kg and placed into the washing machine. The hardness of water was maintained at 24 fH water 2:1, Ca: Mg. 36 g of either Surf Excel QuickWash powder detergent or Persil Non-Bio Liquid detergent was added in the dosing chamber. The swatches were dried using line drying at room temperature (25 °C) for 10 days.

A Konika Minolta spectrophotometer Model-2600d was used to measure the stain intensity on the swatches. L*, a*, b* were measured at three different areas of the stained and untreated fabrics. Δ b* and Δ E* were calculated subsequently.

**Table - 2:**

| Example | Surfactant | INCI name | Surfactant (wt%) | HLB | Δb* |
|---|---|---|---|---|---|
| A | - | | - | - | 3.89 |
| B | Brij S20 | Steareth-20 | 2 | 15 | 2.75 |
| 1 | EO3:EO7 (1:1) | tri(oxyethylene) dodecyl ether: hepta(oxyethylene) dodecyl ether | 2 | 10 | 1.44 |
| 2 | Brij L4 | Polyethylene glycol dodecyl ether | 2 | 9 | 0.68 |
| 3 | Brij S2 | Ceteth-2 | 2 | 5.3 | 1.27 |

The data in the table above shows that the composition comprising a non-ionic surfactant having HLB less than 11 delivers much better whitening as evidenced by the low Δb* value as compared to a composition having high HLB (>11) surfactant or no surfactant at all.

### Example C - E, 4: Whitening obtained with other non-ionic surfactants in experiments done with four wash cycles

Experiments similar to that in Table 2 above were conducted with other surfactants and with four wash cycles. The data is summarised in Table - 3 below:

**Table - 3**

| Example | Surfactant | Surfactant (wt%) | HLB | Δb* |
|---|---|---|---|---|
| C | - | - | - | 4.54 |
| D | Brij 58 | 2 | 16 | 2.78 |
| E | EO3:EO7 (1:1) + Brij 58 | 2 | 13 | 2.32 |
| 4 | EO3:EO7 (1:1) | 2 | 10 | 1.19 |

### The INCI name for Brij 58 is Ceteth-20

The data in the table - 3 above, also confirms the same finding that composition comprising a non-ionic surfactant having HLB less than 11 delivers much better stain removal as compared to a composition having high HLB (>11) surfactant or no surfactant at all.

### Example F-H, 5 - 7: Whitening obtained with combination of non-ionic surfactants at various ratios in experiments done with three wash cycles

Experiments similar to that in Table 3 above were conducted with certain surfactants at various ratios with three wash cycles. The data is summarised in Table - 4 below:

**Table - 4**

| Example | Surfactant | Surfactant (wt%) | HLB | Δb* |
|---|---|---|---|---|
| F | - | - | - | 3.70 |
| G | EO7 | 2 | 12.0 | 2.00 |
| H | EO3:EO7 (1:3) | 2 | 11.0 | 2.53 |
| 5 | EOS | 2 | 8.2 | 1.60 |
| 6 | EO3:EO7 (1:1) | 2 | 10.0 | 1.24 |
| 7 | EO3:EO7 (3:1) | 2 | 9.12 | 1.74 |

The data in the table - 4 confirms the same finding as in the previous tables.

### Example J, 8-10: Whitening obtained with combination of non-ionic surfactants at various concentrations in experiments done with two wash cycles

Experiments similar to that in Table 4 above were conducted with combination of surfactants at various concentrations with two wash cycles. The data is summarised in Table - 5 below:

**Table - 5**

| Example | Surfactant | Surfactant (wt%) | HLB | Δb* |
|---|---|---|---|---|
| J | - | - | - | 3.89 |
| 8 | EO3:EO7 (1:1) | 0.5 | 10.1 | 2.82 |
| 9 | EO3:EO7 (1:1) | 1.0 | 10.1 | 1.95 |
| 10 | EO3:EO7 (1:1) | 2.0 | 10.1 | 1.44 |

The data in the table - 5 above indicates that inclusion of combination of non-ionic surfactants at various concentrations gives desired whitening, where higher concentration in the range of 1-2 wt% is preferred.

Examples K-N and 11: Whitening obtained with the anhydrous composition of the invention in comparison to a high-water containing composition.

A high water containing roll-on composition was prepared as shown in Table - 6 below:

**Table - 6**

| Ingredient | wt% |
|---|---|
| Aluminium chlorohydrate | 12.0 |
| Sunflower seed oil | 2.0 |
| Silica dimethyl silylate | 0.7 |
| Tinogard TT | 0.05 |
| Perfume | 1.0 |
| Water | To 100 |
| Nonionic surfactant, Type | See Table - 7 below |

The following compositions were prepared using base formulation of Table - 1 (Aerosol, anhydrous composition) or base formulation of Table - 6 (Water containing roll-on composition). The whitening delivered by each of the compositions was measured as already mentioned above and the Δb* data is summarised in Table - 7 below:

**Table - 7:**

| Example | Composition | Surfactant (wt%) | Δb* |
|---|---|---|---|
| K | As in Table -1 | - | 5.38 |
| L | As in Table - 6 | - | (**) |
| M | As in Table - 6 + Brij S2 | 2.6 | 2.46 |
| N | As in Table - 6 + Brij S2 | 5.2 | 2.27 |
| 11 | As in Table - 1 + Brij S2 | 2.6 | 1.97 |

| | | | |
|---|---|---|---|
| (**) The formulation was unstable due to the absence of a surfactant in a water and oil containing formulation. The surfactant is necessary to stabilise the emulsion. | | | |

The data in Table - 7 above indicates that the invention is effective only for an anhydrous composition (Example - 11) but is not effective for a water containing composition (Examples M and N).

## Claims

1. An antiperspirant composition comprising
(a) A metal based antiperspirant active;
(b) A natural oil that comprises a glyceride of an unsaturated carboxylic/fatty acid;
(c) An antioxidant compound;
(d) A non-ionic surfactant having an HLB of less than 11; and
(e) less than 10 wt% water.
wherein the non-ionic surfactant is selected from polyoxyethylene sorbitan alkyl esters, fatty alcohol ethoxylates, or sorbitan monoester, alkyl polyglucosides or C8-C16 fatty alcohol glycoside.

2. An antiperspirant composition as claimed in claim 1 wherein the non-ionic surfactant is selected from polyethylene glycol dodecyl ether, Steareth-2, tri(oxyethylene) dodecyl ether or a combination of tri(oxyethylene) dodecyl ether and hepta (oxyethylene) dodecyl ether.

3. An antiperspirant composition as claimed in any one of the preceding claims wherein the non-ionic surfactant is included in 0.5 wt.% to 5 wt.% by weight of the composition.

4. An antiperspirant composition as claimed in any one of the preceding claims wherein said metal based antiperspirant active is selected from an aluminium, zirconium or mixed aluminium/zirconium salt, preferably, aluminium chlorohydrate, aluminium-zirconium tetrachlorohydrex glycine complex, aluminium-zirconium octachlorohydrex glycine complex, aluminium-zirconium pentachlorohydrate, aluminium sesquichlorohydrate or mixtures thereof.

5. An antiperspirant composition as claimed in claim 4 wherein said metal based antiperspirant active is aluminium chlorohydrate, aluminum sesquichlorohydrate or mixtures thereof.

6. An antiperspirant composition as claimed in any one of the preceding claims comprising 1 wt.% to 50 wt.% metal based antiperspirant active by weight of the composition.

7. An antiperspirant composition as claimed in any one of the preceding claims wherein said natural oil is selected from at least one of coriander seed oil, borage seed oil, evening primrose oil, maize corn oil, sunflower oil, safflower oil, coconut oil, and algal oil.

8. An antiperspirant composition as claimed in claim 7 wherein said natural oil is sunflower oil, algal oil or coconut oil preferably sunflower oil.

9. An antiperspirant composition as claimed in any one of the preceding claims comprising 0.1 wt.% to 10 wt.% natural oil by weight of the composition.

10. An antiperspirant composition as claimed in any one of the preceding claims wherein the antioxidant is selected from butyl hydroxy toluene, pentaerythrityl tetra-di-t-butyl hydroxyhydrocinnamate (sold as Tinogard TT), or dilauryl thiodipropionate (sold as Tinogard DA).

11. An antiperspirant composition as claimed in any one of the preceding claims comprising 0.01 wt.% to 2 wt.% antioxidant compound by weight of the composition.

12. An antiperspirant composition as claimed in any one of the preceding claims which is delivered in a stick form, or in an aerosol can.

13. A method of minimizing staining or yellowish coloration of fabric worn by an individual comprising the steps of (a) applying a composition as claimed in any one of the preceding claims on to a body part preferably the axilla of the individual followed by (b) washing, (c) rinsing and (d) drying the fabric.

## Patentansprüche

1. Schweißhemmende Zusammensetzung, umfassend
(a) einen schweißhemmenden Wirkstoff auf Metallbasis;
(b) ein natürliches Öl, das ein Glycerid einer ungesättigten Carbonsäure/Fettsäure enthält;
(c) eine antioxidative Verbindung;
(d) ein nicht-ionisches Tensid mit einem HLB-Wert von weniger als 11; und
(e) weniger als 10 Gew.-% Wasser,
wobei das nicht-ionische Tensid unter Polyoxyethylensorbitanalkylestern, Fettalkoholethoxylaten oder Sorbitanmonoester, Alkylpolyglucosiden oder C8-C16-Fettalkoholglycosid ausgewählt ist.

2. Schweißhemmende Zusammensetzung, wie in Anspruch 1 beansprucht, wobei das nicht-ionische Tensid unter Polyethylenglycoldodecylether, Steareth-2, Tri(oxyethylen)dodecylether oder einer Kombination von Tri(oxyethylen)dodecylether und Hepta(oxyethylen)dodecylether ausgewählt ist.

3. Schweißhemmende Zusammensetzung, wie in irgendeinem der vorhergehenden Ansprüche beansprucht, wobei das nicht-ionische Tensid mit 0,5 Gew.-% bis 5 Gew.-%, bezogen auf das Gewicht der Zusammensetzung, einbezogen ist.

4. Schweißhemmende Zusammensetzung, wie in irgendeinem der vorhergehenden Ansprüche beansprucht, wobei der schweißhemmende Wirkstoff auf Metallbasis unter Aluminium-, Zirconium- oder gemischtem Aluminium/Zirconium-Salz, vorzugsweise Aluminiumchlorhydrat, Aluminium-Zirconium-Tetrachlorhydrex-Glycin-Komplex, Aluminium-Zirconium-Octachlorhydrex-Glycin-Komplex, Aluminium-Zirconium-Pentachlorhydrat, Aluminium-Sesquichlorhydrat oder Mischungen davon ausgewählt ist.

5. Schweißhemmende Zusammensetzung, wie im Anspruch 4 beansprucht, wobei der schweißhemmende Wirkstoff auf Metallbasis Aluminiumchlorhydrat, Aluminiumsesquichlorhydrat oder Mischungen davon ist.

6. Schweißhemmende Zusammensetzung, wie in irgendeinem der vorhergehenden Ansprüche beansprucht, umfassend 1 Gew.-% bis 50 Gew.-% schweißhemmenden Wirkstoff auf Metallbasis, bezogen auf das Gewicht der Zusammensetzung.

7. Schweißhemmende Zusammensetzung, wie in irgendeinem der vorhergehenden Ansprüche beansprucht, wobei das natürliche Öl unter mindestens einem der folgenden Öle ausgewählt ist: Koriandersamenöl, Borretschsamenöl, Nachtkerzenöl, Maiskeimöl, Sonnenblumenöl, Safloröl, Kokosnussöl und Algenöl.

8. Schweißhemmende Zusammensetzung, wie im Anspruch 7 beansprucht, wobei das natürliche Öl Sonnenblumenöl, Algenöl oder Kokosnussöl ist, vorzugsweise Sonnenblumenöl.

9. Schweißhemmende Zusammensetzung, wie in irgendeinem der vorhergehenden Ansprüche beansprucht, umfassend 0,1 Gew.-% bis 10 Gew.-% natürliches Öl, bezogen auf das Gewicht der Zusammensetzung.

10. Schweißhemmende Zusammensetzung, wie in irgendeinem der vorhergehenden Ansprüche beansprucht, wobei das Antioxidans unter Butylhydroxytoluol, Pentaerythrit-tetra-di-t-butylhydroxyhydrocinnamat (verkauft als Tinogard TT) oder Dilaurylthiodipropionat (verkauft als Tinogard DA) ausgewählt ist.

11. Schweißhemmende Zusammensetzung, wie in irgendeinem der vorhergehenden Ansprüche beansprucht, umfassend 0,1 Gew.-% bis 2 Gew.-% antioxidative Verbindung, bezogen auf das Gewicht der Zusammensetzung.

12. Schweißhemmende Zusammensetzung, wie in irgendeinem der vorhergehenden Ansprüche beansprucht, wobei diese in Stabform oder in einer Sprühdose bereitgestellt wird.

13. Verfahren zum Minimieren von Flecken oder von Gelbfärbung auf Textilien, die von einer Person getragen werden, umfassend die Schritte (a) Auftragen einer Zusammensetzung, wie in irgendeinem der vorhergehenden Ansprüche beansprucht, auf einen Körperteil, vorzugsweise auf die Achselhöhle der Person, gefolgt von (b) Waschen, (c) Spülen und (d) Trocknen des Textils.

## Revendications

1. Composition antitranspirante comprenant
(a) Un agent actif antitranspirant à base de métal ;
(b) Une huile naturelle qui comprend un glycéride d'un acide carboxylique/gras insaturé ;
(c) Un composé antioxydant ;
(d) Un tensioactif non ionique ayant un HLB de moins de 11 ; et
(e) moins de 10 % en poids d'eau.
dans laquelle le tensioactif non ionique est choisi parmi des esters alkyliques de polyoxyéthylène sorbitan, des éthoxylates d'alcool gras, ou un monoester de sorbitan, des polyglucosides d'alkyle ou un glycoside d'alcool gras en C8-C16.

2. Composition antitranspirante selon la revendication 1 dans laquelle le tensioactif non ionique est choisi parmi l'éther dodécylique de polyéthylène glycol, le Stéareth-2, l'éther dodécylique de tri(oxyéthylène) ou une combinaison d'éther dodécylique de tri(oxyéthylène) et d'éther dodécylique d'hepta (oxyéthylène).

3. Composition antitranspirante selon l'une quelconque des revendications précédentes dans laquelle le tensioactif non ionique est inclus à raison de 0,5 % en poids à 5 % en poids par poids de la composition.

4. Composition antitranspirante selon l'une quelconque des revendications précédentes dans laquelle ledit agent actif antitranspirant à base de métal est choisi parmi un sel d'aluminium, de zirconium ou mixte d'aluminium/zirconium, de préférence, un chlorhydrate d'aluminium, un complexe aluminium-zirconium tétrachlorohydrex glycine, un complexe aluminium-zirconium octachlorohydrex glycine, un pentachlorhydrate d'aluminium-zirconium, un sesquichlorhydrate d'aluminium ou des mélanges de ceux-ci.

5. Composition antitranspirante selon la revendication 4 dans laquelle ledit agent actif antitranspirant à base de métal est du chlorhydrate d'aluminium, du sesquichlorhydrate d'aluminium ou des mélanges de ceux-ci.

6. Composition antitranspirante selon l'une quelconque des revendications précédentes comprenant 1 % en poids à 50 % en poids d'agent actif antitranspirant à base de métal par poids de la composition.

7. Composition antitranspirante selon l'une quelconque des revendications précédentes dans laquelle ladite huile naturelle est choisie parmi au moins l'une de l'huile de graines de coriandre, de l'huile de graines de bourrache, de l'huile d'onagre, de l'huile de maïs, de l'huile de tournesol, de l'huile de carthame, de l'huile de noix de coco et de l'huile d'algue.

8. Composition antitranspirante selon la revendication 7 dans laquelle ladite huile naturelle est de l'huile de tournesol, de l'huile d'algue ou de l'huile de noix de coco, de préférence de l'huile de tournesol.

9. Composition antitranspirante selon l'une quelconque des revendications précédentes comprenant 0,1 % en poids à 10 % en poids d'huile naturelle par poids de la composition.

10. Composition antitranspirante selon l'une quelconque des revendications précédentes dans laquelle l'antioxydant est choisi parmi le butyl hydroxy toluène, l'hydroxyhydrocinnamate de pentaérythrityl tétra-di-t-butyle (vendu sous le nom de Tinogard TT), ou le thiodipropionate de dilauryle (vendu sous le nom de Tinogard DA).

11. Composition antitranspirante selon l'une quelconque des revendications précédentes comprenant 0,01 % en poids à 2 % en poids de composé antioxydant par poids de la composition.

12. Composition antitranspirante selon l'une quelconque des revendications précédentes qui est fournie sous une forme de bâtonnet, ou dans une bombe aérosol.

13. Procédé pour réduire au maximum l'apparition de taches ou la coloration jaunâtre d'un tissu porté par un individu comprenant les étapes de (a) application d'une composition selon l'une quelconque des revendications précédentes sur une partie du corps, de préférence les aisselles de l'individu, suivie de (b) lavage, (c) rinçage et (d) séchage du tissu.
